# EUROPEAN PATENT APPLICATION

(11) **EP 3 501 568 A1**
(43) Date of publication of application: **26.06.2019**
(21) Application number: 17208791.8
(22) Date of filing: 20.12.2017
(51) Int. Cl.: A61M 5/142, A61M 5/168

(54) **PORTABLE LIQUID INJECTION DEVICE**

(71) Applicant: Valtronic Technologies (Holding) SA, 1343 Les Charbonnières (CH)
(72) Inventor: LEPPLE-WIENHUES, Albrecht, 25300 Pontarlier (FR)
(74) Representative: Patentanwälte Ruff, Wilhelm, Beier, Dauster & Partner mbB

(57) **Abstract**

A portable liquid injection device comprises at least one drug reservoir and at least one discharge opening for parenteral injection, in particular subcutaneous injection of at least one drug. This injection device further provides at least one means for monitoring changes of a physical or chemical property in connection with the injection process at the discharge opening or in close proximity to the discharge opening. Preferably said means is a first electrode for monitoring changes in capacitance, in particular via an electric circuitry.

## Description

### FIELD OF THE INVENTION

This invention relates to a portable liquid injection device comprising at least one drug reservoir and at least one discharge opening for parenteral injection, in particular subcutaneous injection of at least one drug.

### BACKGROUND TO THE INVENTION

Many modern drugs require repeated injections since they cannot be taken orally. Examples for these drugs include peptides like insulin and monoclonal antibodies. These drugs are frequently packaged in injectors that can be multiple use injection pens where multiple, preferably multiple different injection doses can be selected and the pen may be disposed after the drug content is used up. Those injectors also can be single use injectors, where a single shot uses the complete amount of drug and the injector is disposed after injection.

All these injection devices are designed for self-injection by patients, or for injection by trained caretakers. While this avoids frequent, expensive and annoying visits of a physician, frequently patients and caretakers are making mistakes when handling these devices during injection. This can result in wrong dosage, ineffective therapy and even dangerous under- and overdosing events.

A frequent mistake e.g. is retracting the needle from the skin before the entire drug volume is injected. The needle may cause discomfort and the subject is therefore eager to remove the needle quickly. However, pen needles are very fine in order to reduce pain and provide therefore a large resistance to the flow of the liquid drug. Furthermore, after pushing an injection button the subject is required to retain the needle in the skin for up to tens of seconds to assure complete injection. This required delay is even longer for drugs like antibodies that are highly viscous in solution.

Another frequent issue may be the acceptance of the therapy in children, e.g. with insulin or growth hormone. Some children, who are supposed to self-inject, avoid puncturing their skin and dispose of the therapeutic shot otherwise.

Another issue is lack of dexterity of elderly patients, who may be handicapped by tremor, reduced acuity of sight and other ailments, thus inadvertently making mistakes that lead to incomplete injection.

WO 2016/020276 A1 discloses a device that capacitively measures the aqueous volume in the drug container. However, this document does not provide for any means to monitor the injection process at the injection site. E.g., by measuring the drug volume in the device it can not be determined if the drug is indeed injected into the body or simply expelled from the injection device.

### OBJECT OF THE INVENTION

The present invention has the object to overcome the above-mentioned limitations of the discussed prior art. The invention should create the possibility for any user of a portable liquid injection device, e.g. a pen-type injection device, to easily determine whether the predetermined or chosen drug amount is indeed injected into the body of the patient. Consequentially, at least a part of the problems mentioned above should be solved, and the compliance problems with self-injection devices should be remedied.

### DESCRIPTION OF THE INVENTION

The above-mentioned object is solved by the portable liquid injection device with the features of independent claim 1. Preferred embodiments of this inventive device are defined and described in the dependent claims. The wording of all claims is hereby incorporated into this description by explicit reference.

The inventive injection device with at least one drug reservoir and at least one discharge opening comprises at least one means for monitoring changes of a physical or chemical property in connection with the injection process at the discharge opening or in close proximity to the discharge opening.

As said means for monitoring are provided at the discharge opening or in close proximity to the discharge opening, they are also in close proximity to the actual injection site for the drug on the body surface. Therefore it is possible to monitor the injection process itself.

In principle, the inventive injection device can have any design which is possible for devices with at least one drug reservoir and at least one discharge opening. Nevertheless, the inventive injection device preferably is a device known as a so-called autoinjector. These autoinjectors are medical devices designed to deliver a dose of a particular drug. An autoinjector can also be in the form of a pen-like injection device.

Mostly, autoinjectors are syringes which are spring-loaded. Such injectors comprise an actuating element, e.g. a button. If the button is pressed, the needle of the syringe is automatically inserted into the body of the patient and the drug is delivered from the drug reservoir or drug container. Such drug reservoirs are made from glass or from plastics.

Normally, the needle tip with the needle outlet is shielded prior to injection by a protecting cap.

Other designs of autoinjectors are possible. In this context there are injectors having shape and size of a smartphone. This design includes a retractable needle, and automated voice instructions are provided to assist the patient in correct use of this autoinjector.

Also known are gas jet autoinjectors which contain a cylinder of pressurized gas. Via this gas a fine jet of liquid drug is injected through the skin without use of a needle.

It is preferred according to the invention that said means is a first electrode for monitoring changes in capacitance. Preferably, these changes in capacitance are to be monitored via an electric circuitry.

The term "capacitance" means a charge movement driven by a voltage over a distance of an insulating, dielectric layer. The insulating layer could consist of e.g. air, dry skin, glass, or plastic. The capacitance change e.g. due to approaching the skin of a subject can be measured with an electric circuitry suited to measure impedance changes using a suitable voltage signal known to a skilled person.

The term "electrode" means at least one electrode, whose capacitance can be measured against a reference electrode or against an array of electrodes.

In these preferred embodiments the invention makes use of the fact that the human body mainly consists of electrically conductive materials. A predominant component of these conductive materials is water with different salt ions in solution. A normally dry skin surface isolates the conductive body from the environment. The resulting electrical capacitance of the human body (against ground) amounts to several µF. This capacitance can e.g. be sensed by touch sensors and touchscreens through an insulating layer, e.g. a glass screen, or an air gap.

As the present invention in the above-mentioned preferred embodiments adds capacitance electrodes to the discharge opening (or close to the discharge opening), the approach of the discharge opening to the skin and the remaining of the discharge opening on the skin or in the skin can be detected as capacitance changes against ground or against a reference electrode. This capacitance change can be caused by a distance change, and/or by a change in the dielectric material in the electric field. While insulators like air, glass, plastic etc. typically have a relative permittivity of 1-4, the dielectric constant **εᵣ** of water is appr. 80 at room temperature. Therefore, moving a mass of water like a body part into the electric field can be sensed as a change in capacitance.

As it will be explained later, this measurement of capacitance can be combined with and referenced to a capacitance measurement at the drug reservoir in order to evaluate the injection process as a whole.

According to the invention, the injection device can be provided for repeated use, i.e. the drug reservoir can contain an amount of drug provided for at least two injections. Further, according to the invention the injection device can be reusable, i.e. the injection device can be used at least twice, after refill e.g. replacing a drug container/drug reservoir.

However, according to the invention it is preferred that the inventive injection device is disposable, i.e. it is provided (only) for single use.

As mentioned above, the inventive injection device comprises at least one discharge opening for (any kind of) parenteral injection. As a consequence, this discharge opening can be any kind of orifice or outlet, e.g. of a pipe or container/chamber between said discharge opening and the drug reservoir.

Nevertheless, it is preferred according to the invention that said discharge opening is the outlet of a hypodermic needle. As explained above, with these embodiments the approach of the outlet of the needle, and consequently the approach of the needle to the skin, can be detected, and later also the penetration of the needle into the skin can be detected, both as capacitance and/or impedance changes against ground or against a reference electrode.

In a first group of further preferred embodiments said means for monitoring changes of a physical or chemical property, in particular said first electrode for monitoring changes in capacitance, is integrated into the injection device. Therefore, e.g. said first electrode is placed inside the injection device at the discharge opening or in close proximity to the discharge opening. If said discharge opening is the outlet of a needle, e.g. said first electrode preferably is placed at the site where the needle is attached to the body of the injection device.

In a second group of further preferred embodiments of the present invention said means for monitoring changes of a physical or chemical property, in particular said first electrode for monitoring changes in capacitance, is part of a device which is attached or attachable to the injection device. Preferably, said attached or attachable device is in the form of a sleeve or in the form of a cap. Such a cap can additionally have the function of protecting a relevant part of the injection device, e.g. the drug reservoir/drug container and/or the needle (before it is used for injection).

Said (attachable or attached) device, preferably said sleeve or cap, can have a lid or cover which can be removed reversibly to open and close the device. For this purpose said lid or cover can be connected to the device with a hinge. In this context it is also possible to provide concentric sliding elements or an iris-type shutter or a sideway tilting cylindric element for protecting e. g. the needle.

The preferred embodiments comprising a sleeve or cap will be explained later in the context of the drawings. Nevertheless, the term "cap" means any component to be attached to an injection device which covers (and normally protects) another critical component or part of the injection device, e.g. the needle for injecting the corresponding drug.

According to the invention said first electrode (being electrically conductive for measuring and monitoring capacitance) can have any (geometrical) form or shape. This electrode preferably can have the shape of a disc or a plate. It is also preferred that said first electrode is ringshaped or has the shape of a ring segment or a cylinder segment. The first electrodes are preferably flat wherein the term "flat" means that these electrodes have a low height or thickness compared to their other two dimensions.

As explained above, e.g. the changes in capacitance can be detected against ground. Nevertheless, it is preferred according to the invention if the injection device comprises at least one second electrode as a reference electrode. In these cases the capacitance changes are detected against this reference electrode. Normally, the inventive injection device will comprise at least one actuating element for starting (and preferably continuing) the injection process, e.g. a push button. As a consequence, it is further preferred according to the invention to provide said reference electrode at the actuating element or in proximity to the actuating element of the injection device.

In another embodiment the reference electrode is located close to a part of the device that is used as a handle during the injection process.

As an alternative said at least one second electrode (reference electrode) can be provided as part of the (additional) device attached or attachable to the injection device, preferably the sleeve or cap as described above.

In other preferred embodiments of the invention the injection device comprises at least one third electrode. This at least one third electrode is assigned to the drug reservoir for detecting the filling level of the drug reservoir. In particular, said at least one third electrode is provided for monitoring said filling level during the injection process by measuring changes in capacitance via an electric circuitry.

As an alternative said at least one third electrode can be provided as part of the (additional) device attached or attachable to the injection device, preferably the sleeve or cap as described above.

As already discussed above, these last mentioned embodiments offer the possibility to reference the capacitive measurement at the first electrodes (in close proximity to the injection site) to a capacitive measurement of the third electrodes at the drug reservoir. This measure allows the analysis of the relative timing of the event "discharge opening at the injection site" (e.g. "needle in skin") versus the event "drug ejection started and ongoing". This analysis and the corresponding data can be processed, creating information for the patient or the caretaker, which e.g. can be transmitted e.g. wirelessly. As a consequence, with these preferred embodiments of the invention it can be guaranteed that the necessary amount of drug is indeed transferred from the drug reservoir into the body of the patient.

The above-mentioned preferred embodiments of the invention comprising at least one third electrode assigned to the drug reservoir can be further designed with the following preferred features.

First, at least two electrodes can be assigned to the drug reservoir as third electrodes.

Further, said third electrodes can be flat, elongated electrodes. The term "flat" means that the electrodes, irrespective of their actual shape or design, have a low height or thickness compared to their other two dimensions. Preferably, said flat electrodes have the shape of cylindrical surface segments with a low height or thickness of the actual electrode body.

Further, said third electrodes are designed such that preferably the longest dimension of the electrodes is provided to be parallel with an axis of the drug reservoir.

Further, at least 3, e.g. 3, 5 or 7, third electrodes are provided according to the invention. Preferably, the corresponding number of third electrodes is distributed along the circumference of the drug reservoir in regular distances, e.g. 3 electrodes are located on a cylindrical circumferential surface offset by 120°.

Further, said third electrodes are preferably provided in pairs, in particular along the circumference of the drug reservoir, for forming a capacitor. This results in a total number of 2n third electrodes with n ≥ 1, wherein n preferably is between 1 and 10.

Further, said third electrodes and/or said third electrode pairs can be separately switched for modifying the electrical field to be applied across the drug reservoir in multiple rotational directions perpendicular to an axis of the injection device.

In one of the last mentioned preferred embodiments two pairs of third electrodes are provided at the drug reservoir, wherein at each of the two opposite ends of the drug reservoir one of these two pairs is located. This arrangement allows the determination of the beginning and the end of the outflow of the drug from the reservoir, e.g. of the beginning and also of the end of the movement of a plunger, which is typically used in an inventive injection device to eject the drug from the drug reservoir. As a consequence, it can be detected that the drug ejection from the drug reservoir is complete. This is due to the fact that the third electrode pairs can detect the replacement of the water column between the electrodes by the plunger material.

In an additional preferred embodiment of the inventive injection device an outer electrical shield is provided around said third electrodes assigned to the drug reservoir. Such a shield acts as a Faraday cage shielding the electrodes from external interference and avoids any impedance changes caused by factors located outside of the inventive device.

In this context it is further preferred that the outer electrical shield (around the third electrodes adjacent to the drug reservoir) is (simultaneously) used as a reference (second) electrode.

In further preferred embodiments the inventive injection device further comprises a signaling device, in particular an acoustic signaling device and/or an optical signaling device for indicating a status in connection with the injection process. Preferably, a critical status in connection with the injection process should be indicated. With these embodiments e.g. there is an acoustic or optic feedback for the patient or caretaker, in particular informing them when e.g. the needle can be safely retracted and/or when the injection process was not performed properly. In this context, additionally or alternatively, a log entry can be created to be reviewed later by caretakers or other users.

In preferred embodiments the inventive device comprises a (electric) power source, in particular a battery or an accumulator. Preferably, for holding the power source the inventive device can have a cavity being a compartment for said power source (and perhaps other additional components of the device). In particular, such a compartment has a cover holding the power source in place.

Further, the inventive device preferably comprises a rigid and/or flexible printed circuit board (PCB). Such a PCB can be easily integrated into the inventive device as a substrate for all necessary equipment, e.g. not only including all circuitry, but also e.g. the electrodes on such PCB.

In addition, according to the present invention, it is preferred that the injection device comprises at least one display. E.g. this display can be used to show a status of the injection device, in particular a status related to the injection process to the patient or to the caretaker or even to the physician. This status can be e.g. a critical status as outlined above, and in all these cases the signaling device as discussed above can be coupled to the display.

Further, the inventive device can include at least one of the following additional components, namely:
- at least one USB (Universal Serial Bus) or any other wired communication unit to transfer data to an external computer or mobile calculator, and
- at least one wireless communication unit to transfer data to an external computer or mobile calculator, e.g. Bluetooth.

The inventive device preferably is made of plastic material, in particular of a plastic material formed by injection molding.

The inventive injection device is associated with a number of advantages. The most important advantage is that with the inventive device a reliable statement is possible whether the discharge opening is indeed in contact with the skin of the patient, in particular whether the outlet of the needle has indeed penetrated into the skin and remains in the skin until injection is completed.

Preferably, with the inventive device the approach of the discharge opening, in particular the outlet of the needle to the skin, and separately the above-mentioned penetration of the needle into the skin can be detected, preferably as a capacitance change against ground or against a reference electrode.

Thus, the entire process of injection and its timing can be closely monitored, and handling errors can be identified. An information feedback can then be provided to the patient and/or to the caretaker in order to remedy the problem by training or by supervision.

In preferred embodiments of the inventive injection device the capacitive measurement of a first electrode (as defined above) can be referenced to a capacitive measurement of third electrodes (as defined above) assigned to the drug reservoir. This reference of "skin proximity electrodes" to "drug reservoir electrodes" allows the analysis of the relative timing of the event "discharge opening on the skin/needle in the skin" versus the event "ejection of the drug from the drug reservoir ongoing". In other words: With these preferred embodiments a control is possible whether the drug was indeed ejected from the drug reservoir during the period of contact between discharge opening and skin (preferably needle in the skin). With these embodiments it is also possible to check whether the necessary amount of drug was ejected from the reservoir during the injection process (or whether the drug reservoir is empty after the injection process (single-use injection device)).

Further advantages and features of the invention will become clear from the following description of the drawings and the example. The individual features can be realized either singly or jointly in combination in one embodiment of the invention. The drawings and the example only serve for illustration and better understanding of the invention and are not to be understood as in any way limiting the invention.

The drawings schematically show:
- Fig. 1: a first embodiment of the inventive injection device with a first electrode integrated into the device,
- Fig. 2A to Fig. 2D: a second embodiment of the inventive injection device with a first electrode integrated into a cap which can be attached to the injection device (in different arrangements), and
- Fig. 3: two different embodiments of the arrangement of third electrodes assigned to the drug reservoir of an inventive injection device.

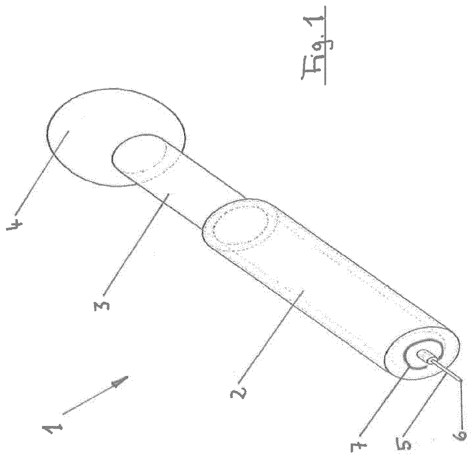

Fig. 1 shows an inventive injection device 1 in a schematic representation.

The main components of injection device 1 are (main) body 2 housing a drug reservoir/drug container (not shown), a handle 3 that may contain a spring and a plunger (not shown), an actuating element 4, and needle 5 with its outlet 6. By actuating and pressing element 4 the drug contained in body 2 is ejected through needle 5 into the body of the patient. A protecting element for the needle before use of injection device 1 is not shown in Fig. 1.

Further, Fig. 1 shows a first electrode 7 in the shape of a ring. This electrode 7 is close to needle 5 and to its outlet 6, and therefore close to the injection site during the injection process. Therefore, changes in capacitance can be detected and monitored, e.g. against ground or against at least one second electrode (reference electrode), which can preferably be provided in proximity to actuating element 4. Second electrodes, an electric circuitry, and also third electrodes assigned to the drug reservoir in body 2 are not shown in Fig. 1.

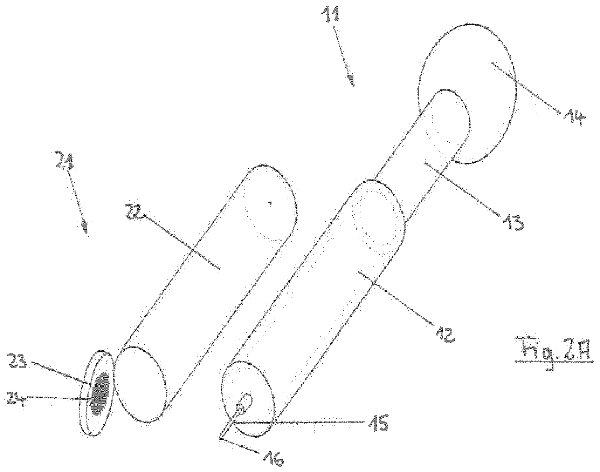

In Fig. 2A another injection device 11 according to the invention is schematically shown. Further, Fig. 2A shows a cap 21 in schematic representation, which can be attached to injection device 11.

As shown in Fig. 2A, injection device 11 also comprises a (main) body 12 housing a not-shown drug reservoir, a handle 13, an actuating element 14, and a needle 15 with its outlet 16.

However, in contrast to injection device 1 shown in Fig. 1, injection device 11 does not include a first electrode.

As shown in Fig. 2A, a first electrode 24 for detecting and monitoring changes in capacitance at the injection site during the injection process is provided at the cap-like attachment device 21.

This cap 21 comprises a sleeve-like body 22 and a cover or lid 23. This lid 23 can be in a closed and opened position. For this purpose part 22 and part 23 can be connected via a suitable fastening element like a hinge (not shown). The lid 23 is shown here as a hinged lid as a preferred embodiment. As mentioned earlier, other preferred embodiments enclose and protect the needle by concentric sliding elements or by an iris-type shutter or by a sideway tilting cylindric element (not shown).

According to the embodiment of the invention shown in Fig. 2A, a first electrode 24 close to the injection site in the form of a disc-like element is integrated into lid 23. With the aid of this electrode 24 changes in capacitance can be detected and monitored against ground or against a reference electrode (second electrode).

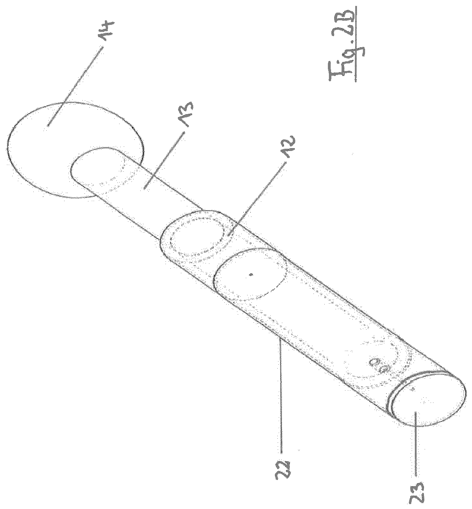

In Figs. 2B to Fig. 2D different arrangements of the embodiment shown in Fig. 2A for preparing the injection process with injection device 11 and its cap 21 are shown.

According to Fig. 2B cap 21 with its sleeve-like body 22 and its (closed) lid 23 is placed on injection device 11 with its body 12 and its actuating element 14. For this purpose, the diameter of body 22 is a little bit larger than the diameter of body 12. When cap 21 is placed on injection device 11, needle 15 of injection device 11 is protected by cap 21.

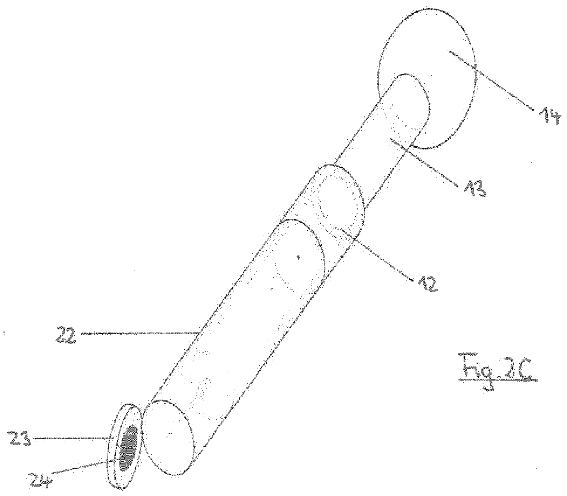

Fig. 2C shows the status of the arrangement according to Fig. 2B if an injection process is being prepared. In this status lid 23 opens while body 22 of cap 21 is still on body 12 of injection device 11. If lid 23 opens, needle 15 (not shown in Fig. 2C) is exposed, as well as first electrode 24 on lid 23.

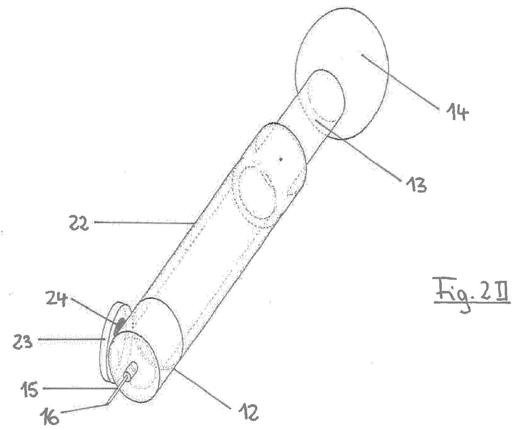

The next step in the preparation of an injection process with injection device 11 and cap 21 is shown in Fig. 2D. Here, cap 21 with body 22 and lid 23 slides backwards on injection device 11 with body 12, handle 13 and actuating element 14. Now needle 15 with its outlet 16 is in injection position. First electrode 24 on lid 23 is still in close proximity to needle 15 for detecting and monitoring changes in capacitance at the injection site.

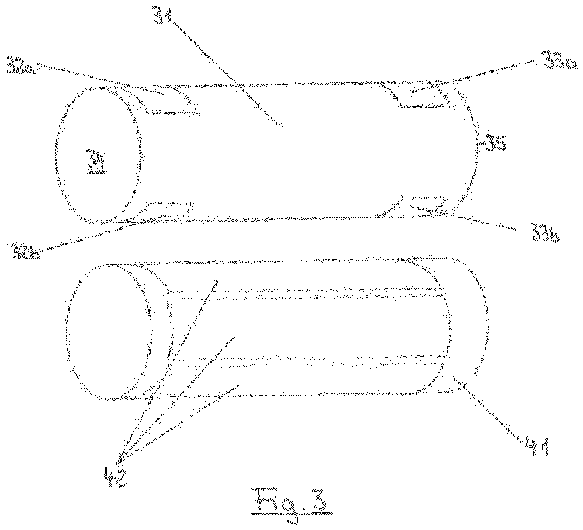

Fig. 3 shows two different embodiments how third electrodes can be arranged on a drug reservoir in order to sense the filling state of this drug reservoir (representation is also schematic).

The upper embodiment of Fig. 3 shows a drug reservoir 31 with two pairs of third electrodes 32a, 32b and 33a, 33b, respectively, at the two opposite ends 34 and 35 of drug reservoir 31.

Third electrodes 32a, 32b, 33a, 33b are flat conductive elements along the circumference of reservoir 31 with an offset of 180°.

With the arrangement of this upper embodiment of Fig. 3 it is possible to detect the presence of the plunger in the syringe-like injection device and consequently the beginning and also the end of the outflow of the drug from reservoir 31.

In the lower embodiment of Fig. 3 a drug reservoir 41 and third electrodes 42 are shown. Electrodes 42 are in the shape of flat conductive stripes on the circumference of reservoir 41 along the longitudinal axis of reservoir 41. In this lower embodiment of Fig. 3 six third electrodes 42 in the form of stripes are arranged on reservoir 41 with an offset of 60°.

With this latter embodiment not only the beginning and the end of the outflow of the drug from reservoir 41 can be detected, but the whole ejection process of the drug can be monitored.

### EXAMPLES

An inventive injection device as shown in Fig. 1 is used for injecting a liquid drug into the body of a patient. For this purpose the corresponding injection device does not only include a first electrode for detecting and monitoring changes in capacitance in close proximity to the injection site, but also a second electrode as a reference electrode in proximity to the actuating element and also third electrodes assigned to the drug reservoir. As a consequence, changes in capacitance can be measured close to the needle (with the "needle electrode"), close to the actuating element (with the "push button electrode") and close to the drug reservoir (with the "drug container electrodes").

In the examples two kinds of injection processes are simulated, namely a "good injection" and an "incomplete injection". A "good injection" is an injection process in which the user carefully approximates the needle to the skin, penetrates the skin and keeps the needle in the skin during the whole ejection process of the drug from the drug reservoir. In an "incomplete injection" e.g. the user removes the needle from the skin before the entire drug volume is injected.

These two cases, namely "good injection" and "incomplete injection" are shown in the two representations below.

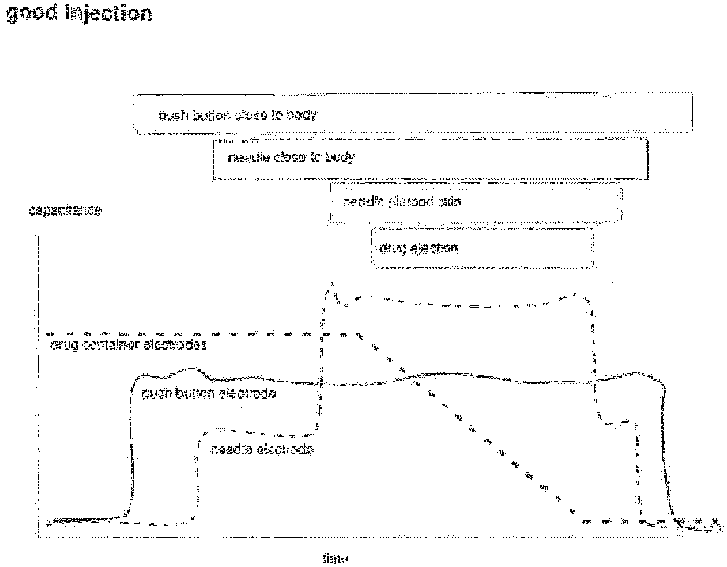

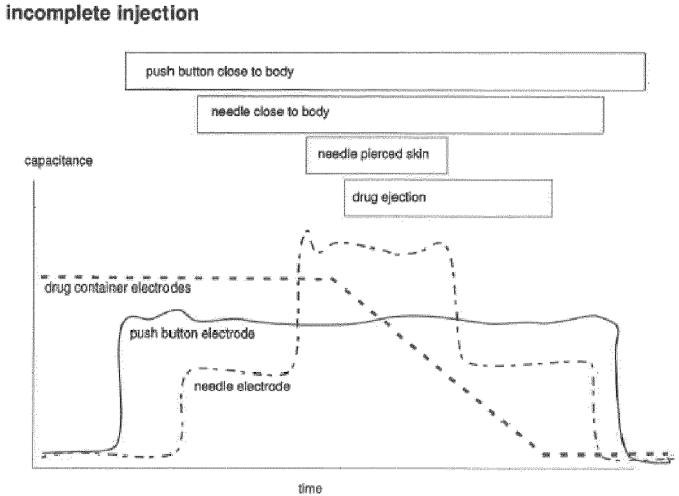

In both representations capacitance is plotted as a function of time, whereby the values of the needle electrode, the push button electrode and the drug container electrodes are shown over time with different dash patterns.

In both representations the capacitance of the needle electrode is increasing in a first step, when the needle approaches the skin at the injection site. Then, when the needle perforates the skin, the capacitance of the needle electrode is increasing again sharply and remains constant as long as the needle stays embedded in the skin. This increase in the capacitance of the needle electrode is reversed upon removal of the needle from the skin. More generally, it is of note that other impedance measurements may reveal a decrease of impedance when perforating the skin, that will then indicate the "needle embedded in skin" phase.

The capacitance of the push button electrode increases as soon as the fingers of the user approach the actuating element. This value of capacitance is constant as long as there is contact between the fingers of the user and the actuating element. If the fingers are removed from the actuating element the capacitance of the push button electrode decreases.

The capacitance measured by the drug container electrodes remains a constant as long as no drug is ejected from drug reservoir. As soon as drug is ejected from the reservoir the capacitance of the drug container electrodes declines until the reservoir is empty.

Based on these facts the two representations for a "good injection" and an "incomplete injection", respectively can be explained.

With a "good injection" the capacitance plateau of the needle electrode corresponds (and overlaps) with the declining part of the capacitance of the drug container electrodes. This means that the needle obviously remained in the skin as long as drug was ejected from the drug reservoir.

In contrast, with an "incomplete injection", there is no correspondence between the capacitance plateau of the needle electrode and the declining part of the capacitance of the drug container electrodes. Obviously the needle was removed from the skin before the full content of the drug reservoir was ejected. Therefore, the injection was not correctly performed and incomplete.

E.g. such an incorrect injection (incomplete injection) can be communicated to the user or caretaker, e.g. at the end of the injection process, or even during the injection process. In the latter case an optical or acoustic signal could be provided as soon as the capacitance of the needle electrode decreases in order to communicate that obviously the needle was removed from the skin, and a log entry could be created to be reviewed later by caretakers or users.

## Claims

1. Portable liquid injection device (1;11) comprising at least one drug reservoir and at least one discharge opening for parenteral injection, in particular subcutaneous injection of at least one drug, wherein at least one means for monitoring changes of a physical or chemical property in connection with the injection process is provided at the discharge opening or in close proximity to the discharge opening .

2. Injection device according to claim 1 **characterized in that** said means is a first electrode (7;24) for monitoring changes in capacitance, in particular through an electric circuitry.

3. Injection device according to claim 1 or claim 2, **characterized in that** said injection device is a disposable injection device.

4. Injection device according to anyone of the preceding claims, **characterized in that** said discharge opening is the outlet (6;16) of a needle (5;15).

5. Injection device according to anyone of the preceding claims, **characterized in that** said means, in particular said first electrode, is integrated into the injection device.

6. Injection device according to anyone of claims 2 to 5, further comprising at least one second electrode as a reference electrode, wherein preferably said reference electrode is provided at an actuating element or at a handle or in proximity to an actuating element or to a handle of the injection device.

7. Injection device according to anyone of claims 2 to 6, further comprising at least one third electrode (32,33;42), preferably assigned to the drug reservoir (31;41), for detecting the filling level of the drug reservoir, in particular for monitoring said filling level during the injection process by measuring changes in capacitance via an electric circuitry.

8. Injection device according to claim 7 **characterized in that** at least two electrodes are provided as third electrodes.

9. Injection device according to claim 7 or claim 8 **characterized in that** said third electrodes are flat, elongated electrodes, wherein preferably the longest dimension of the electrodes is provided to be parallel with an axis of the drug reservoir.

10. Injection device according to anyone of claims 7 to 9 **characterized in that** said third electrodes are provided in pairs, in particular along the circumference of the drug reservoir, for forming a capacitor, resulting in a total number of 2 n third electrodes with n ≥ 1, wherein n preferably is between 1 and 10.

11. Injection device according to anyone of claims 7 to 10, **characterized in that** said third electrodes and/or said third electrode pairs can be separately switched for modifying the electrical field.

12. Injection device according to anyone of claims 7 to 11 **characterized in that** an outer electrical shield is provided around said third electrodes assigned to the drug reservoir.

13. Injection device according to claim 12 **characterized in that** said electrical shield is used as a reference electrode.

14. Injection device according to anyone of claims 1 to 4 and claims 6 to 13 **characterized in that** said means, in particular said first electrodes, and/or said second electrodes and/or said third electrodes are part of a device attached or attachable to the injection device, wherein preferably said attached or attachable device is in the form of a sleeve or in the form of a cap (21).

15. Injection device according to anyone of the preceding claims, further comprising a signaling device, in particular an acoustic signaling device and/or optical signaling device for indicating a status, in particular a critical status in connection with the injection process.
